# EUROPEAN PATENT SPECIFICATION

(11) **EP 1 409 650 B1**
(45) Date of publication and mention of the grant of the patent: **12.04.2006**
(21) Application number: 02750989.2
(22) Date of filing: 29.05.2002
(51) Int. Cl.: C12N 5/08, A61K 35/14

(54) **EX-VIVO ISOLATED CD25+CD4+ T CELLS WITH IMMUNOSUPPRESSIVE ACTIVITY AND USES THEREOF**
EX-VIVO ISOLIERTE CD25+CD4+ T ZELLEN MIT IMMUNSUPPRESSIVER AKTIVITÄT UND DEREN ANWENDUNGEN
LYMPHOCYTES T CD25+CD4+ ISOLES EX VIVO A ACTIVITE IMMUNOSUPPRESSIVE ET UTILISATIONS

(30) Priority: 30.05.2001 US 294030 P
(43) Date of publication of application: 21.04.2004
(73) Proprietor: Fondazione Telethon, 00161 Roma (IT)
(72) Inventor: RONCAROLO, Maria, Grazia, I-20132 Milano (IT); LEVINGS, Megan, I-20132 Milano (IT)
(74) Representative: Banchetti, Marina
(86) International application number: PCT/EP2002/005919
(87) International publication number: WO 2002/097070

(56) References cited:
- STEPHENS LEIGH A ET AL: "Human CD4+CD25+ thymocytes and peripheral T cells have immune suppressive activity in vitro." EUROPEAN JOURNAL OF IMMUNOLOGY, vol. 31, no. 4, April 2001 (2001-04), pages 1247-1254, XP002210570 ISSN: 0014-2980
- RONCAROLO M-G ET AL: "The role of different subsets of T regulatory cells in controlling autoimmunity" CURRENT OPINION IN IMMUNOLOGY, CURRENT BIOLOGY LTD, XX, vol. 12, no. 6, 1 December 2000 (2000-12-01), pages 676-683, XP004257742 ISSN: 0952-7915
- LEVINGS MEGAN K ET AL: "Human CD25+CD4+ T regulatory cells suppress naive and memory T cell proliferation and can be expanded in vitro without loss of function." JOURNAL OF EXPERIMENTAL MEDICINE, vol. 193, no. 11, 4 June 2001 (2001-06-04), pages 1295-1301, XP002210571 ISSN: 0022-1007

## Description

The present invention provides ex-vivo isolated human CD25⁺CD4⁺ T regulatory (Tr) cells, homogeneous clonal populations derived therefrom with enhanced suppressive activity and their uses in the regulation of immune responses and for the identification and characterization of suppressor T cell specific molecules. More specifically, the invention is directed to the use of polyclonal CD25⁺CD4⁺ Tr cells or of homogeneous clonal CD25⁺CD4⁺ Tr cells generated ex-vivo to prevent or treat conditions where a down-regulation/suppression of the immune response is required, such as graft-vs-host disease (GvHD), organ rejection, gene therapy and autoimmune diseases, or for the identification and characterization of molecules involved in the regulation of the immune response.

### BACKGROUND OF THE INVENTION

T regulatory (Tr) cells have a key role in the maintenance of immune tolerance to both self and harmless foreign antigens. Many subsets of Tr cells have been described and recently much progress has been made in understanding their ontogeny, function and mechanisms of action (reviewed in (1)). Some Tr cells do not produce cytokines and suppress T-cell responses via a mechanism that requires direct cell-cell contact (2, 3). Other subsets of Tr cells produce immunoregulatory cytokines, such as IL-10 and TGF-β, and exert their suppressive functions at least in part via the effects of these cytokines (4-8).

CD4⁺ Tr cells that constitutively express the IL-2R α chain (CD25) have been identified in the mouse (reviewed in (2, 3)). These CD25⁺CD4⁺ Tr cells show a remarkable suppressive capacity both *in vitro* and *in vivo.* Transfer of these Tr cells reduces the pathology of experimentally-induced autoimmune diseases such as thyroiditis, gastritis, insulin-dependent diabetes mellitus and colitis.(9-12) and of experimentally induced GvHD (31), whereas depletion of CD25⁺CD4⁺ Tr cells results in the development of systemic autoimmune diseases (11, 13, 14).

Murine CD25⁺CD4⁺ Tr cells are anergic when stimulated *in vitro* with anti-CD3 mAbs, but proliferate upon addition of exogenous IL-2 (15, 16). After TCR-mediated stimulation, CD25⁺CD4⁺ Tr cells suppress the activation and proliferation of other CD4⁺ and CD8⁺ T cells in an antigen non-specific manner (16, 17) via a mechanism that requires cell-cell contact and that, in most systems, is independent of production of immunosuppressive cytokines (15, 16). Murine CD25⁺CD4⁺ Tr cells constitutively express cytotoxic T lymphocyte-associated antigen 4 (CTLA-4) (9), a negative regulator of T-cell activation, and expression of this molecule is required for the ability of these cells to suppress immune responses *in vivo* (10, 18). In addition, CD25⁺CD4⁺ Tr cells may act by down-regulating the expression of CD80 and CD86 on APCs (19), although some reports suggest that APCs are not required for their suppressive activity and indicate that direct T-T cell interaction is involved (17).

### DESCRIPTION OF THE INVENTION

This invention is based on the findings that human CD25⁺CD4⁺ Tr cells with immunosuppressive effects can be isolated from peripheral blood and expanded *in vitro* without loss of function, and that human CD25⁺CD4⁺ Tr cells constitute a heterogenous population from which different cell clones exhibiting suppressive or non-suppressive activity can be derived and isolated based on expression of CD25. Isolated human CD25⁺CD4⁺ Tr cells and CD25⁺CD4⁺ Tr cell clones can be used as immunosuppressive agents for the prevention or treatment of pathologies where a reduction of the immune response is desired. Typically, they will be used to prevent GvHD, organ rejection, immune responses directed against foreign proteins introduced during gene therapy and autoimmune diseases, especially type 1 diabetes. CD25⁺CD4⁺ Tr cells isolated from peripheral blood can be stimulated and cultured in vitro, allowing for the possibility to select and expand antigen-specific suppressor T cells. Expanded CD25⁺CD4⁺ Tr cells maintain their regulatory capacity in vitro, and thus can be used to regulate T cell responses in vitro, whereas both freshly-isolated and in vitro-expanded human CD25⁺CD4⁺ Tr cells can be utilized in therapy in vivo. The methods and conditions for isolation and in vitro expansion of CD25⁺CD4⁺ Tr cells are described in detail in the section Materials and Methods. Essentially, freshly-isolated human CD25⁺CD4⁺ Tr cells can be expanded in vitro under one or more of the following conditions: co-culture with feeder-cell mixture, polyclonal stimulation, antigen specific stimulation, addition of cytokines.

The T cells thus obtained can be re-introduced in the patient. The preferred modalities under which CD25⁺CD4⁺ Tr cells are used in therapy or prophylaxis will depend on the particular condition to prevent/treat.

For example, to prevent/treat GvHD, CD25⁺CD4⁺ Tr can be isolated from leukapheresis of the bone-marrow donor, frozen if necessary, and administered to the recipient at the time of transplant, prior to the transplant, or in the subsequent months. Alternatively, CD25⁺CD4⁺ Tr cells from the donor could be stimulated with host APC in vitro, in order to generate and expand alloantigen-specific CD25⁺CD4⁺ Tr cells that would specifically suppress host-specific responses in vivo.

To prevent/treat organ rejection, CD25⁺CD4⁺ Tr cells can be isolated from the recipient, frozen if necessary, and administered prior to transplant, at the time of transplant or in the subsequent months. Alternatively, CD25⁺CD4⁺ Tr cells could be stimulated in vitro with autologous APCs that have been co-cultured with tissue from the organ in question and will therefore present foreign antigens through the indirect pathway (32). The resulting CD25⁺CD4⁺ Tr cell lines would be specific for antigens expressed by the transplanted organ and could be used to suppress organ-specific responses in vivo.

To prevent autoimmune diseases, bulk populations of autologous CD25⁺CD4⁺ Tr cells can be isolated and re-infused. Alternatively, antigen-specific CD25⁺CD4⁺ Tr cells could be expanded in vitro by stimulation with autologous APCs and self-antigens derived from tissues which are targets of the disease. Upon re-administration of the in vitro-expanded CD25⁺CD4⁺ Tr cells, they will suppress anti-self responses in vivo.

To prevent immune response in gene therapy, CD25⁺CD4⁺ Tr cells can be isolated from the recipient, and cells which are specific for antigens encoded by the therapeutic vector could be expanded in vitro by stimulation with transduced autologous APCs expressing the transgene. These cells can be frozen if necessary, and administered at the time of gene therapy treatment or in the subsequent months.

Advantageously, a homogeneous clonal population of CD25⁺CD4⁺ Tr suppressive cells is used for the therapeutic applications indicated above. The method for isolating suppressive CD25⁺CD4⁺ Tr clones essentially comprises the steps of:
a) purifying CD4⁺ T cells from PBMCs;
b) separating CD25⁺ from CD25⁻ T cells;
c) cloning CD25⁺CD4⁺ T cells by limiting dilution;
d) stimulating with phytohemagglutinin (PHA) or anti-CD3 mAb in the presence of IL-2;
e) selecting the cell clones that display a constitutively high expression of CD25.

According to step (a), CD4⁺ cells can be purified by positive selection with anti-CD4-coupled microbeads. Step (b) can be carried out by marking CD25⁺ cells using labelled anti-CD4/25 monoclonal antibodies and purifying CD25⁺ cells by FACS-sorting. The clones obtained from step (c), which can be maintained in X-vivo 15 culture-medium or in other cellular media, supplemented with 5% pooled or autologous human serum, are preferably restimulated by co-culture with feeder-cell mixture, by antigens or by cytokines, more preferably by an allogenic or autologous feeder-cell mixture consisting of irradiated PBMCs, with or without irradiated autologous or allogeneic EBV-transformed cell lines (eg. JY). Suppressive clones which display a constitutively high expression of CD25 can be selected, according to step (d), on the basis of the following characteristics:
- 100% constant-positivity for CD25 expression in the resting phase at least 10 days after stimulation with phytohemagglutinin or anti-CD3 mAb in the presence of IL-2;
- expression of CD25 at a significantly higher level in comparison to T cell clones isolated in parallel from CD25⁻CD4⁺ T cells or non suppressive clones isolated from CD25⁺CD4⁺ T cells.

At the end of steps (a)-(d), homogeneous CD25⁺CD4⁺ T-cell clones constitutively expressing CD25, anergic and with high suppressive capacity are isolated. The suppressive clones, in contrast to the non-suppressive ones, are characterized by significant production of TGF-β and no production of IL-2.

In a further embodiment, the invention provides an immunosuppressive agent containing isolated CD25⁺CD4⁺⁻Tr-cells and/or CD25⁺CD4⁺ Tr cell clones constitutively expressing CD25, and optionally other active substances, such as cytokines, or other immunosuppressive proteins. Preferably the immunosuppressive agent will be in the form of a stabilized cell preparation.

Besides the envisaged clinical applications, the CD25⁺CD4⁺ Tr suppressive cell clones can be used to set up systems in vitro for the identification of molecules that modulate the immune response, in particular suppressor-T-cell-specific molecules. According to a preferred embodiment, such CD25⁺CD4⁺ Tr cell clones will be used in large scale gene expression arrays, in differential proteomics screenings and in the generation of monoclonal antibodies specific for Tr cells constitutively expressing CD25. These applications are greatly aided by the homogeneity of comparative samples, such as that provided by cell populations of clonal origin.

### DESCRIPTION OF THE FIGURES

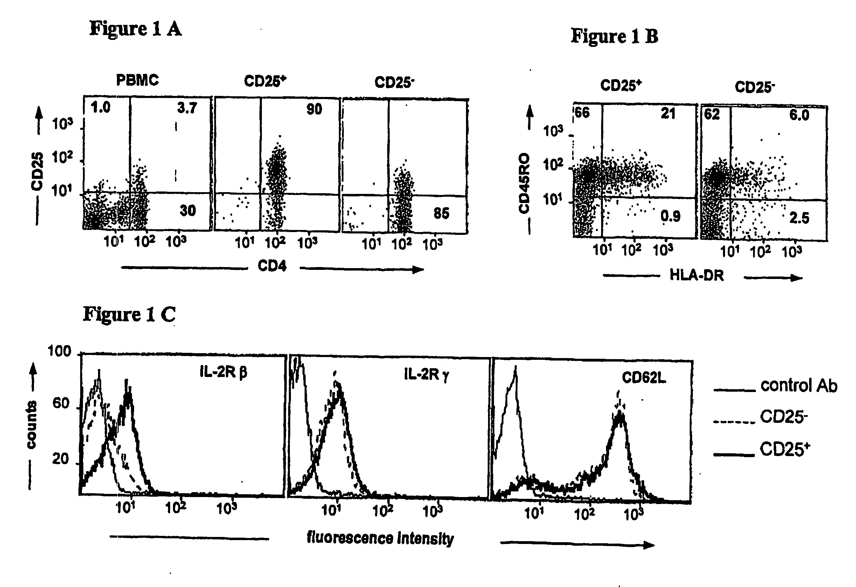

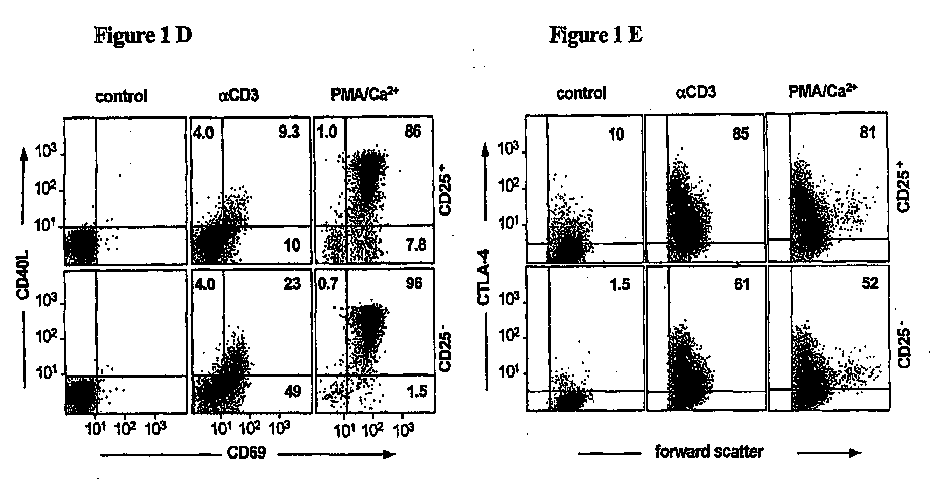

**Figure 1.** *Isolation and cell-surface phenotype of human CD25*^{*+*}*CD4*^{*+*} *Tr cells.* CD4⁺ T cells were isolated from PBMCs, and separated into CD25⁺ and CD25⁻ fractions. Purity (**A**) and expression of CD45RO, HLA-DR (**B**), IL-2Rβ, IL-2Rγ and CD62L (**C**) was determined by FACS. CD25⁺CD4⁺ and CD25⁻CD4⁺ T cells were either cultured in medium alone, or activated with immobilized anti-CD3 mAbs or PMA and calcium ionophore for 6 hours (**D**) or 24 hours (**E**). Cells were analyzed for surface-expression of CD40L and CD69 (**D**), and for intracytoplasmic expression of CTLA-4 (**E**). Results are representative of 6 independent experiments.

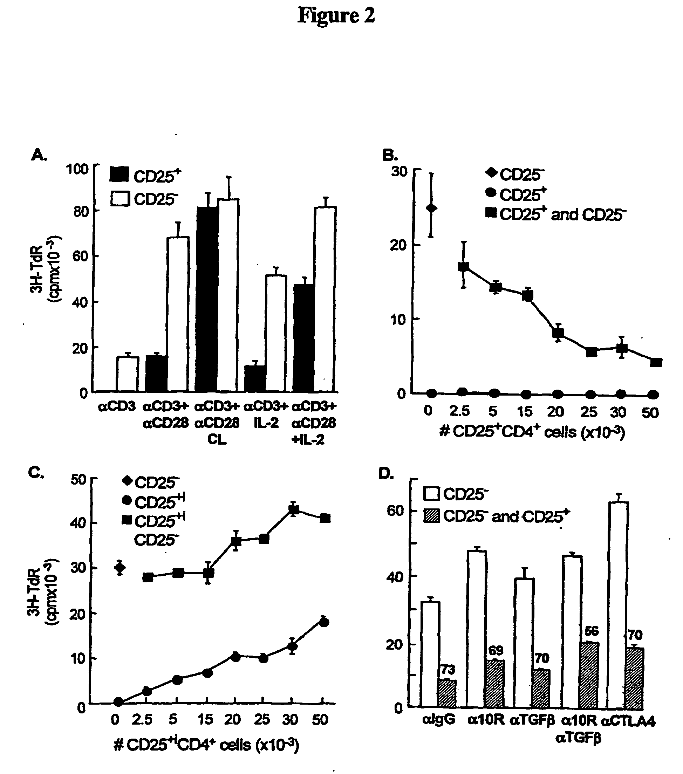

**Figure 2.** *CD25*^{*+*}*CD4*^{*+*} *Tr cells are anergic and suppress proliferation to alloantigens.* Purified CD25⁺CD4⁺ Tr cells (100,000 cells/well) were tested for their ability to proliferate in response to immobilized anti-CD3 mAbs **(αCD3)** (10µg/ml) in the absence or presence of soluble anti-CD28 mAbs **(αCD28)** (1µg/ml), secondary rabbit anti-mouse Abs **(αCD28 CL)** (10µg/ml), and/or IL-2 (100U/ml). After 72 hours of culture, ³H-thymidine was added for an additional 16 hours (**A**). CD25⁻CD4⁺ T cells (50,000 cells/well) were tested for their ability to proliferate in response to allogeneic APCs in the absence or presence of increasing numbers of autologous CD25⁺CD4⁺ Tr cells (**B**). CD25⁻CD4⁺ T cells were activated to induce CD25 expression. After 48 hours T cells that became CD25⁺ (**CD25**⁺ⁱ) were purified and tested for their ability to suppress proliferation of CD25⁻ T cells in response to alloantigens (C). CD25⁻CD4⁺ T cells were activated by alloantigens with or without CD25⁺CD4⁺ Tr cells (1:1 ratio) in the presence of the indicated mAbs (10µg/ml). Numbers represent the percent reduction in proliferation compared to culture in the absence of CD25⁺CD4⁺ Tr cells (**D**). For **B-D,** after 96 hours, ³H-thymidine was added for an additional 16 hours. Results are representative of 6 independent experiments for **A**, 9 for **B** and 3 for **C&D**.

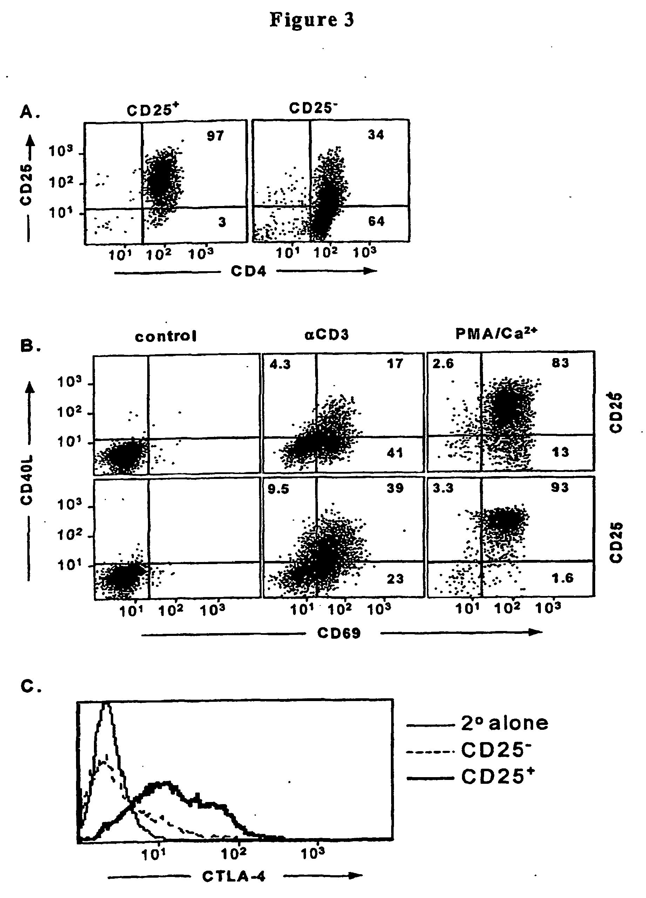

**Figure 3.** *Expansion and cell-surface phenotype of CD25*⁺*CD4*⁺ *Tr cells.* CD25⁺ and CD25⁻CD4⁺ T cells were purified, and activated with anti-CD3 mAbs, allogeneic feeder-cell mixture and exogenous IL-2. Cells were split as necessary and after 2 weeks were analyzed by FACS for expression of CD25 and CD4 (**A**). In parallel, cells were analyzed for cell-surface expression of CD40L and CD69 following activation for 6 hours with immobilized anti-CD3 mAbs or PMA and calcium ionophore (**B**). Constitutive levels of CTLA-4 expression was determined by intracytoplasmic staining (**C**). Results are representative of 3 independent experiments.

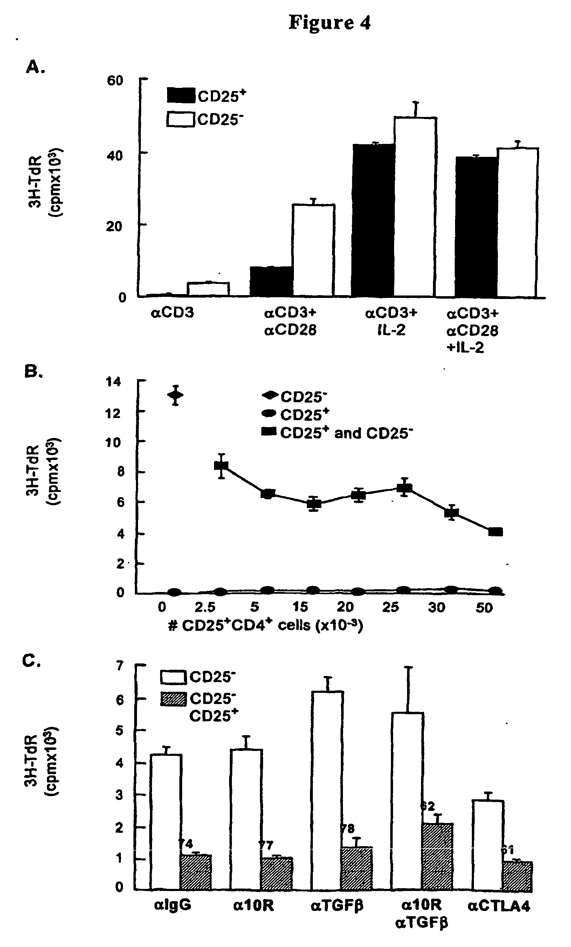

**Figure 4.** *Cultured CD25*^{*+*}*CD4* ⁺ *Tr cells retain their suppressive capacity*. CD25⁺ and CD25⁻CD4⁺ T cells were purified and activated with anti-CD3 mAbs, allogeneic feeder-cell mixture and exogenous IL-2. After 14 days of culture, T cells were tested for their ability to proliferate in response to anti-CD3 mAbs (10µg/ml) in the absence or presence of soluble anti-CD28 mAbs (1µg/ml) and/or IL-2 (100U/ml) (**A**). Cultured CD25⁻CD4⁺ T cells (50,000 cells/well) were tested for their ability to proliferate in response to alloantigens in the absence or presence of increasing numbers of *in vitro-*cultured, autologous CD25⁺CD4⁺ Tr cells (**B**). Cultured CD25⁻CD4⁺ T cells were activated by allogeneic APCs (from a donor different from that used for expansion) with or without CD25⁺CD4⁺ Tr cells (1:1 ratio) in the presence of the indicated mAbs (10µg/ml). Numbers represent the percent reduction in proliferation compared to culture in the absence of CD25⁺CD4⁺ Tr cells (**C**). For all cultures, after 48 hours, ³H-thymidine was added for an additional 16 hours. Results are representative of 3 independent experiments.

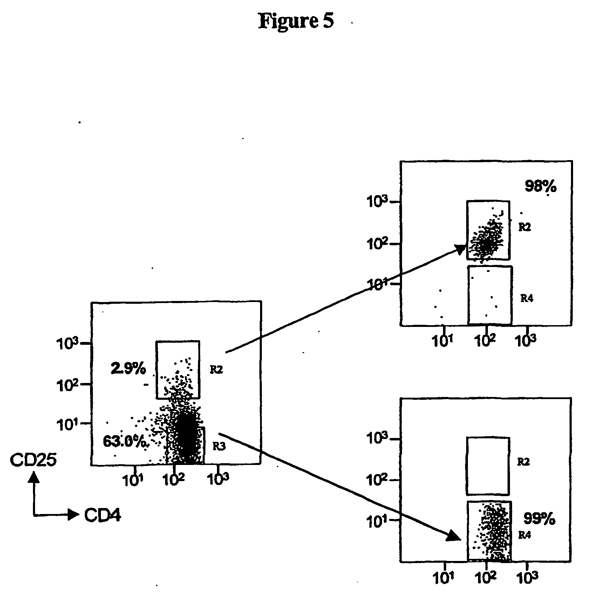

**Figure 5.** *Isolation of human CD25*⁺*CD4*⁺ *T cells at the clonal level*. CD4⁺ T cells were isolated from peripheral blood, stained with anti-CD4 and anti-CD25 mAbs, and separated into CD25⁺CD4⁺ and CD25⁻CD4⁺ T cells by FACS sorting to a purity greater than 98 and 99% respectively.

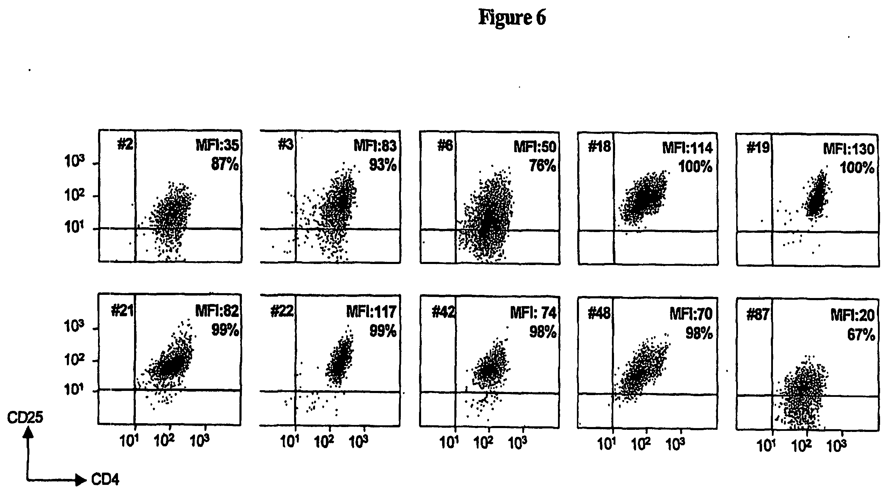

**Figure 6.** *CD25*^{*+*}*CD4*^{*+*} *T cell clones are heterogenous in terms of their expression of CD25 in the resting phase.* Resting T-cell clones were stained with anti-CD4 and -CD25 mAbs 12-14 days after the last re-stimulation, The number (#) of the T cell clone is indicated on the upper left, and the MFI and percent of CD25 positive cells is on the upper right.

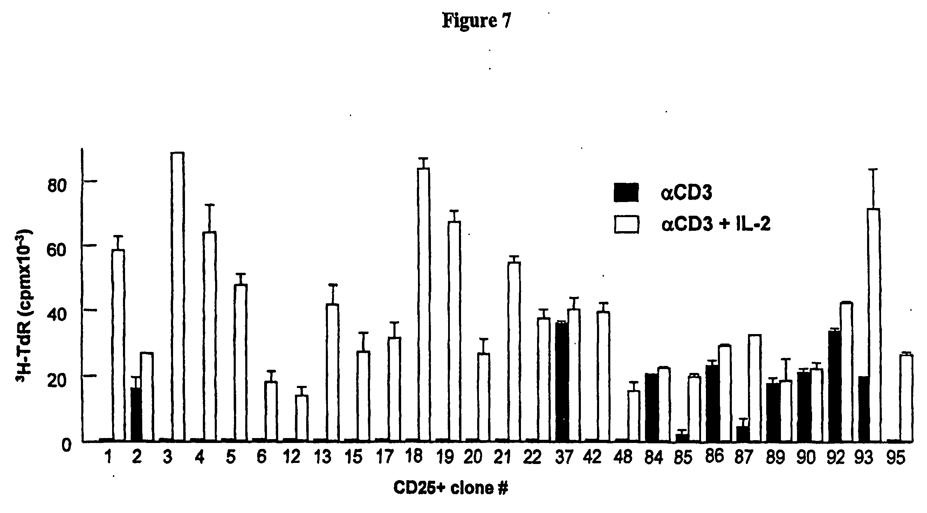

**Figure 7.** *CD25*^{*+*}*CD4*^{*+*} *T cell clones are heterogenous in term of their proliferative response to activation via the TCR.* Resting T-cell clones were tested for their ability to proliferate in response to anti-CD3 mAbs (10µg/ml) in the absence or presence of IL-2 (100U/ml). After 48 hours of culture, ³H-thymidine was added for an additional 16 hours.

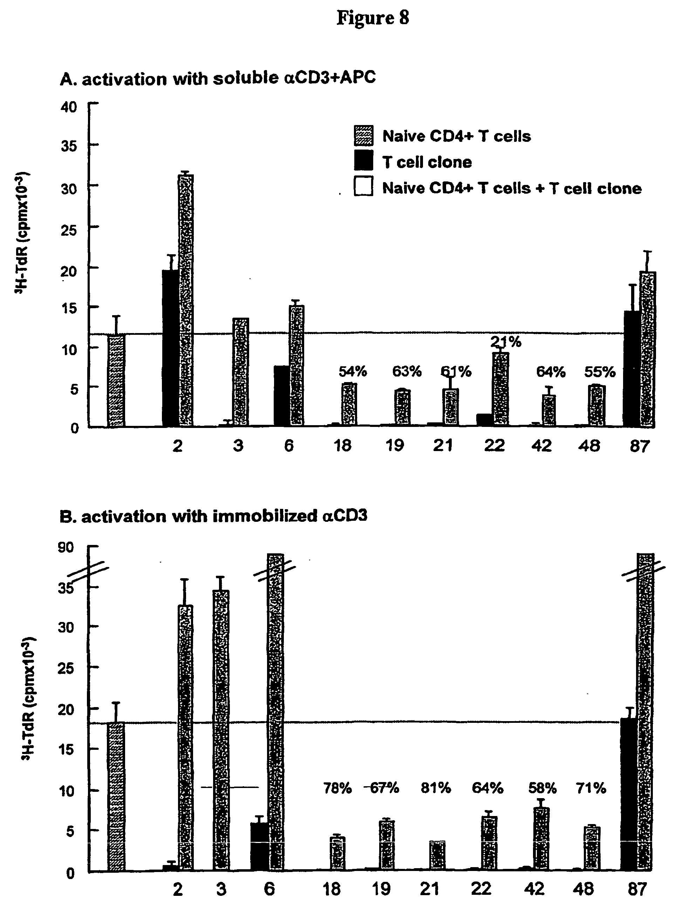

**Figure 8.** *Suppression of naive T cell responses by CD25*^{*+*}*CD4*^{*+*} *T cell clones.* Autologous CD4⁺ T cells were purified and tested for their ability to proliferate in response to anti-CD3 mAbs and irradiated CD3-depleted APCs (**A**) or anti-CD3 mAbs immobilized on plastic (**B**). After 72 (**A**) or 48 hours (**B**) of culture, ³H-thymidine was added for an additional 16 hours. Numbers indicate percent reduction in proliferation in comparison to the naive CD4⁺ T cells alone.

### DETAILED DESCRIPTION OF THE INVENTION

### Isolation and cell-surface phenotype of human CD25⁺CD4⁺ Tr cells

CD25⁺CD4⁺ Tr cells are present in human PBMCs. On average they represent 3.0% (range 1.6-4.4%, n=6) of total PBMCs and 12.8% (range 9.8-18.1 %, n=6) of CD4⁺ T cells. These cells could be readily isolated, with purities greater than 90% (Figure 1A). The majority (82 ±5.1%) of freshly isolated CD25⁺CD4⁺ Tr cells also expressed CD45RO and the percentage of CD25⁺CD4⁺ Tr cells expressing HLA-DR was significantly higher than that in the CD25⁻CD4⁺ population (17.3 ±4.9% vs 6.4 ±2.9%, n=6) (Figure 1B). In addition, human CD25⁺CD4⁺ Tr cells expressed higher levels of the IL-2Rβ in comparison to CD25⁻CD4⁺ T cells (61.9 ±2.6% vs 33.1 ±2.5%, n=5) (Figure 1C) and a subset of freshly isolated CD25⁺CD4⁺ Tr cells expressed CTLA-4 (8.4 ±1.6%, n=6) (Figure 1E). In contrast, expression of the IL-2Rγ and CD62L was equivalent on both populations of T cells. CD25⁺CD4⁺ Tr cells and CD25⁻CD4⁺ T cells were CD3⁺TCRαβ⁺. Thus, human CD25⁺CD4⁺ Tr cells express markers which are characteristic of memory T cells and low constitutive levels of CTLA-4, similarly to murine CD25⁺CD4⁺ Tr cells (9, 10, 15, 17, 18).

Following TCR-mediated stimulation, human CD25⁺CD4⁺ Tr cells expressed lower levels of activation markers in comparison to CD25⁻CD4⁺ T cells. The proportion of CD40L+ cells was 17.3 ±2.3% in CD25⁺CD4⁺ Tr cells vs 28.4 ±1.8% in the CD25⁻CD4⁺ T-cell subset (p 0.005); whereas 30.9 ±7.0% of CD25⁺CD4⁺ Tr cells vs 54 ±9.1% of CD25⁻CD4⁺ T cells expressed CD69 (p 0.006) (Figure 1D). Time course experiments demonstrated that the reduced levels of CD40L and CD69 on CD25⁺CD4⁺ Tr cells were not due to altered kinetics of expression. After activation with PMA and calcium ionophore there was no statistically significant difference between expression of CD69 or CD40L on CD25⁺CD4⁺ or CD25⁻CD4⁺ T cells, although in general fewer CD25⁺CD4⁺ Tr cells expressed CD40L.

Following activation with anti-CD3 mAbs or PMA and calcium ionophore, the percentage of CD25⁺CD4⁺ Tr cells expressing CTLA-4 was higher than that of CD25⁻CD4⁺ T cells. In addition, CTLA-4 expression levels were approximately 3 fold higher on CD25⁺CD4⁺ Tr cells (Figure 1E). Collectively, these data demonstrate that upon TCR activation human CD25⁺CD4⁺ Tr cells have a surface molecule expression profile which is unique and distinct from that of other CD4⁺ T-cell subsets.

### Proliferation and cytokine production by human CD25⁺CD4⁺ Tr cells

Freshly isolated CD25⁺CD4⁺ Tr cells did not proliferate in response to immobilized anti-CD3 mAbs, and addition of soluble anti-CD28 mAbs resulted in a modest and variable increase in proliferation. In contrast, crosslinked anti-CD28 mAbs completely reversed the unresponsiveness of CD25⁺CD4⁺ Tr cells to TCR activation. Addition of exogenous IL-2 partially restored the proliferation of CD25⁺CD4⁺ Tr cells in response to anti-CD3 mAbs, and proliferation was further enhanced by soluble anti-CD28 mAbs (Figure 2A). These results indicate that human CD25⁺CD4⁺ Tr cells have a specific defect in their ability to proliferate after TCR-mediated activation (15, 16).

Human CD25⁺CD4⁺ Tr cells were analyzed for their ability to produce cytokines. Following stimulation with immobilized anti-CD3 mAbs, with or without soluble anti-CD28 mAbs, no detectable levels of IL-2, IL-10, IL-4, TGF-β or IFN-γ could be measured. In contrast, when stimulated with anti-CD3 and soluble anti-CD28 mAbs in the presence of exogenous IL-2, CD25⁺CD4⁺ Tr cells produced significant levels of IL-4, IL-10, IFN-γ and TGF-β (Table 1). Under these stimulation conditions CD25⁺CD4⁺ Tr cells had a cytokine profile that was comparable to that of CD25⁻CD4⁺ T cells. In contrast, differences in cytokine production were observed following activation with allogeneic APCs. Both CD25⁺CD4⁺ and CD25⁻CD4⁺ T cells produced IL-10, TGF-β and IFN-γ, but no IL-4. However, the striking difference between the CD25⁺CD4⁺ and CD25⁻CD4⁺ T cell populations is that the CD25⁺ cells failed to secrete IL-2, indicating that these cells have a specific defect in production of IL-2. Interestingly, CD25⁺CD4⁺ Tr cells consistently produced less IFN-γ upon alloantigen stimulation than did CD25CD4⁺ T cells.

### Human CD25⁺CD4⁺ Tr cells suppress the proliferative responses of naive CD25⁻CD4⁺ T cells to alleantigens.

We investigated the regulatory properties of CD25⁺CD4⁺ Tr cells by testing their ability to suppress the proliferative responses of naive CD25⁻CD4⁺ T cells to alloantigens. CD25⁻CD4⁺ T cells were stimulated with allogeneic APCs and increasing numbers of autologous CD25⁺CD4⁺ Tr cells were added. Addition of as few as 2,500 CD25⁺CD4⁺ Tr cells to 50,000 CD25⁻CD4⁺ T cells resulted in a reduced proliferation of CD25⁻CD4⁺ T cells. At a ratio of 1:1, CD25⁺CD4⁺ Tr cells inhibited the proliferation of naive CD25⁻CD4⁺ T cells by an average of 75.0 ±2.9% (n=9) (Figure 2B). The CD25⁺CD4⁺ Tr cells themselves failed to proliferate in response to alloantigens. Furthermore, CD25⁺CD4⁺ Tr cells suppressed the proliferation of CD25⁻CD4⁺ T cells in response to PHA in the presence of APCs (by an average of 81.2 ±5.0%, n=6) and to immobilized anti-CD3 alone (by an average of 79.4 ±14.6%, n=3). These latter data indicate that CD25⁺CD4⁺ Tr cells have a direct suppressive effect on T cells that is independent of APCs, similar to murine CD25⁺CD4⁺ Tr cells (17). In addition, CD25⁺CD4⁺ Tr cells suppressed production of IFN-γ and IL-2 by CD25⁻CD4⁺ T cells activated with anti-CD3 mAbs or allogeneic APCs.

In order to demonstrate that this suppressive capacity was an intrinsic property of T cells constitutively expressing CD25 in vivo, we tested whether CD25⁻CD4⁺ T cells which expressed CD25 following activation *in vitro* showed regulatory effects. To this aim, CD25⁻CD4⁺ T cells were activated with anti-CD3 and anti-CD28 mAbs, and after 48 hours the CD25⁺ T cells were isolated and tested for their ability to suppress freshly isolated autologous CD25⁻ T cells. As shown in Figure 2C, T cells which became CD25⁺ after *in vitro* activation proliferated in response to alloantigens, and enhanced rather than suppressed the response of CD25⁻CD4⁺ T cells. These data indicate that inhibition of T cell proliferation is a unique property of cells which constitutively express CD25 *in vivo.*

Several studies show that some subsets of Tr cells, such as type 1 Tr (Tr1) and Th3 cells, produce IL-10 and/or TGF-β and suppress immune responses via production of these cytokines (4-8). Since CD25⁺CD4⁺ Tr cells produced both IL-10 and TGF-β upon stimulation with allogeneic APCs (Table 1) the role of these cytokines on inhibition of allogeneic responses by human CD25⁺CD4⁺ Tr cells was investigated. As shown in Figure 2D, addition of neutralizing anti-IL-10R or anti-TGF-β mAbs had no significant effect on the ability of CD25⁺CD4⁺ Tr cells to suppress the proliferation of CD25⁻CD4⁺ T cells in response to alloantigens. In 3 independent experiments an average of 67.2 ±6.0% suppression was observed in the presence of 10µg/ml of control IgG, 63.8 ±5.8% with 10µg/ml of anti-IL-10R and 69.0 ±3.4% with 10µg/ml of anti-TGF-β mAbs. Similar results were obtained with a 5 fold higher concentration of anti-TGF-β mAbs. Addition of both anti-IL-10R and anti-TGF-β mAbs resulted in a slight, but not statistically significant, reversal of suppression mediated by CD25⁺CD4⁺ Tr cells (from 67.2 ±6.0% to 52.4 ±5.7%, p 0.06).

F(ab')₂ fragments from antibodies which specifically block the ability of CTLA-4 to bind to CD80/86, without affecting signals via CD28, have previously been shown to inhibit the production of TGF-β by Tr1 cells (20). Addition of the same blocking anti-CTLA-4 mAbs had no significant effect on the suppressive activity of CD25⁺CD4⁺ Tr cells. These data suggest that despite the fact that CD25⁺CD4⁺ Tr cells express high levels of CTLA-4 (Figure 1E), this molecule is not required for their suppressive activity.

### Human CD25⁺CD4⁺ Tr cells can be expanded in vitro

We have previously shown that human Tr1 cells which have been cloned and expanded *in vitro* maintain their regulatory activity (6). Using a protocol similar to that described for Tr1 cells, we determined whether CD25⁺CD4⁺ Tr cells could be expanded. CD25⁺CD4⁺ Tr cells did not proliferate in response to anti-CD3 alone (Figure 2A), but when activated with anti-CD3 mAbs in the presence of an allogeneic feeder-cell mixture and exogenous IL-2, an expansion which was similar to that of CD25⁻CD4⁺ T cells was obtained (20-30 fold increase at day 14). *In vitro*-expanded human CD25⁺CD4⁺ Tr cells remained positive for CD25 even after culture for more than one month (Figure 3A). In contrast, all CD25⁻CD4⁺ T cells expressed CD25 after activation, but the expression gradually decreased with time. Persistent expression of CD25 has also been observed in murine CD25⁺CD4⁺ Tr cells activated *in vivo* (21).

Similar to freshly isolated CD25⁺CD4⁺ Tr cells, the proportion of *in vitro*-expanded CD25⁺CD4⁺ Tr cells expressing CD40L following activation with anti-CD3 mAbs was consistently lower in comparison to CD25⁻CD4⁺ T cells (12.7 ±3.3% vs 36.9 ±8.3%, p 0.01). However, in contrast to freshly isolated cells, cultured CD25⁺CD4⁺ Tr cells expressed normal levels of CD69 following polyclonal TCR-mediated activation (Figure 3B). Thus, reduced upregulation of CD40L is also a characteristic of expanded CD25⁺CD4⁺ Tr cells. As expected, cultured CD25⁺CD4⁺ and CD25⁻CD4⁺ T cells expressed similar levels of both CD40L and CD69 following activation with PMA and calcium ionophore. *In vitro*-expansion of CD25⁺CD4⁺ Tr cells did not alter their constitutive expression of CTLA-4, and they continued to express this inhibitory molecule at significantly higher levels than their CD25⁻CD4⁺ counterparts (Figure 3C).

### In vitro-expanded CD25⁺CD4⁺ Tr cells remain anergic and retain their suppressive capacity

In vitro-expanded CD25⁺CD4⁺ Tr cells failed to proliferate in response to anti-CD3 mAbs alone, and proliferation could only be completely restored by addition of exogenous IL-2 (Figure 4A). Moreover, cultured CD25⁺CD4⁺ Tr cells failed to proliferate in response to alloantigens, and retained their ability to suppress the proliferation of autologous CD25⁻CD4⁺ T cells (at a 1:1 ratio, an average 64 ±3.8% (n=3) suppression was observed) (Figure 4B). These data indicate that CD25⁺CD4⁺ Tr cells expanded *in vitro* maintain their regulatory functions and behave similarly to freshly isolated CD25⁺CD4⁺ Tr cells. Since in this experiment the CD25⁻CD4⁺ responder T cells had been previously activated and expanded *in vitro,* these data demonstrate that CD25⁺CD4⁺ Tr cells suppress not only the response of freshly isolated naive CD25⁻CD4⁺ T cells, but also that of previously activated memory CD25CD4⁺ T cells (Figure 4B). Finally, similar to observations with freshly isolated CD25⁺CD4⁺ Tr cells, the suppression mediated by in vitro-expanded CD25⁺CD4⁺ Tr cells was not reversed by addition of neutralizing anti-IL-10R, anti-TGF-β or anti-CTLA-4 mAbs (Figure 4C).

In the present study we show that human CD4⁺ T cells which express CD25 in vivo are a unique subset of Tr cells. Human CD25⁺CD4⁺ Tr cells are anergic, fail to produce IL-2, constitutively express CTLA-4, and suppress the proliferation of naive CD4⁺ T cells, as described for murine CD25⁺CD4⁺ Tr cells (2, 3). In addition, following polyclonal TCR-mediated activation, human CD25⁺CD4⁺ Tr cells strongly upregulate CTLA-4, display reduced expression of CD40L, and produce cytokines. CD25 and CTLA-4 remain constitutively expressed on in vitro-expanded human CD25⁺CD4⁺ Tr cells, while following activation, up-regulation of CD40L is still defective. More interestingly, in vitro expanded CD25⁺CD4⁺ Tr cells retain their potent suppressive activity, even towards previously activated memory T cells. The observation that functional Tr cells can be expanded in vitro and can regulate responses of memory T cells is of great clinical relevance for the use of CD25⁺CD4⁺ Tr cells as a cellular therapy in T-cell mediated diseases.

The role of immunoregulatory cytokines in the suppression mediated by CD25⁺CD4⁺ Tr cells remains an open question. Alloantigen-activated CD25⁺CD4⁺ Tr cells did not proliferate but produced IL-10, IFN-γ and TGF-β, and indeed possessed a profile of cytokine production which is comparable to that of Tr1 cells (i.e. IL-10+IFN-γ+TGF-β+IL-4-IL-2-/low) (6). However, we observed only a slight reversal of suppression in the presence of both neutralizing anti-IL-10R and anti-TGF-β mAbs, which is consistent with previous observations that production of IL-10 and TGF-β is dispensable for the regulatory function of CD25⁺CD4⁺ Tr cells (15, 16). On the other hand, in a murine model of experimentally-induced colitis, both IL-10 and TGF-β were found to be required for suppression mediated by CD25⁺CD4⁺ Tr cells (5, 10). The basis for this discrepancy in the involvement of IL-10 and TGF-β is unclear. CD25⁺CD4⁺ Tr cells have the capacity to produce IL-10 and TGF-β, but production of these cytokines may depend on their maturation state and the environmental context in which they are activated.

Previous reports demonstrated that direct cell-cell contact is required for murine CD25⁺CD4⁺ Tr cells to exert their suppressive effects (15, 16). Despite constitutive and persistent expression of CTLA-4, anti-CTLA-4 mAbs failed to abrogate the suppressive activity of human CD25⁺CD4⁺ Tr cells. These data are in agreement with a study indicating that signals via CTLA-4 were dispensable for suppression by mouse CD25⁺CD4⁺ Tr cells in vitro (15). However, more recent reports indicate that expression of CTLA-4 is essential for suppression mediated by these cells (10, 18). It is possible that suppression of proliferation operates via mechanisms which differ depending on the stimuli and microenvironment, or that human and mouse CD25⁺CD4⁺ Tr cells act through different mechanisms. Finally, suppression is not simply due to consumption of IL-2 as murine CD25⁺CD4⁺ Tr cells suppressed IL-2 production at the transcriptional level (15). In addition, human CD4⁺ T cells which expressed CD25 following activation in-vitro did not suppress responses of CD25⁻CD4⁺ T cells, indicating that high expression of CD25 does not simply result in sequestration of IL-2.

Human CD25⁺CD4⁺ Tr cells expand in vitro and maintain their unique cell-surface marker profile and suppressive functions. To our knowledge, these data represent the first report of in vitro expansion of human T suppressor cell lines.

The clinical use of CD25⁺CD4⁺ T regulatory (Tr) cells can be envisaged to down-regulate undesired immune responses in a number of pathological conditions. We have shown that CD25⁺CD4⁺ Tr cells with suppressive function can be readily isolated from peripheral blood. Importantly, these cells can be stimulated and cultured in vitro, allowing for the possibility to select and expand antigen-specific suppressor T cells. Expanded CD25⁺CD4⁺ Tr cells maintain their regulatory capacity in vitro, and thus could be used to regulate T cell responses in vitro.

### Isolation and characterization at the clonal level of human CD25⁺CD4⁺ T cells with suppressive capacity

In order to determine the relationship between IL-10 producing Tr1 cells (22) and CD25⁺CD4⁺ Tr cells (23, 24), we attempted to isolate CD25⁺CD4⁺ Tr cells at the clonal level. It has previously been described that only approximately 0.8% of peripheral blood mononuclear cells which are CD4⁺ and high for CD25⁺ have a suppressive capacity in vitro (25). We therefore purified CD4⁺ T cells from peripheral blood, and by FACS sorting, purified CD25^{bright}CD4⁺ Tr cells which represent approximately 2.9% of CD4⁺ T cells (0.6% of total peripheral blood in this donor). The resulting CD25⁻ and CD25^{bright} populations were 99 and 98% pure respectively (Figure 5). The purified cells were subsequently cloned by limiting dilution at 1 cell/well and stimulated with PHA and an allogeneic feeder cell mixture as described in the materials and methods and as previously described (26) After 8 days, one 96-well plate from each cloning was pulsed overnight with thymidine in order to determine the number of proliferating wells (the cloning efficiency). The CD25⁻CD4⁺ T cells had a cloning efficiency of 42%, whereas the CD25⁺CD4⁺ T cells had a significantly lower efficiency of only 10.2%. After 14 days, 120 CD25⁺CD4⁺ T-cell clones were picked, restimulated and expanded for analysis.

As one of the defining characteristics of CD25⁺CD4⁺ Tr cells is constitutive and high expression of CD25, we screened the CD25⁺CD4⁺ T-cell clones for expression of CD25 at least 10 days after restimulation (in the resting phase). A shown in Figure 6, the clones displayed a heterogeneous expression of CD25. Approximately half the clones remained 99-100% positive for CD25, and possessed a relatively high mean fluorescence intensity (MFI). Other clones contained a significant number of CD25⁻ cells and a lower MFI. T-cell clones derived the CD25⁻CD4⁺ T cells displayed a low percentage of CD25⁺ cells in the resting phase and consequently also a low MFI.

The clones were subsequently tested for their ability to proliferate in response to activation via the TCR in the absence or presence of exogenous IL-2. It has been well established that both murine and human CD25⁺CD4⁺ T cells fail to proliferate in response to αCD3 mAbs in the absence of costimulation via CD28 and/or addition of IL-2 (23, 24, 26). In order to determine if this was also true at the clonal level, we tested the CD25⁺CD4⁺ T-cell clones for their ability to proliferate in the presence or absence of IL-2. Similar to the heterogeneity observed in terms of expression of CD25, the clones were also heterogeneous in terms of proliferation. The majority of the clones (58/72, 80%) were anergic and failed to proliferate in response to αCD3 mAbs, but proliferated well in the presence of IL-2. The remaining clones (14/72, 20%) proliferated well in response to αCD3 mAbs even in the absence of IL-2. A representative subset of the 72 cloned tested is shown in Figure 7. In contrast, amongst the CD25⁻CD4⁺ T-cell clones tested, the majority (14/22, 65%) proliferated well in response to αCD3 mAbs alone.

The heterogeneity of CD25⁺CD4⁺ T-cell clones in terms of expression of CD25 and proliferation suggested that even within the 0.6% CD25^{bright}CD4⁺ Tr cell population of PBMCs, not all cells may be suppressor cells, and that a proportion could be activated T helper cells. To test this hypothesis we performed in vitro suppression assays. As shown in Figure 8, indeed only a subset of the CD25⁺CD4⁺ T cell clones were able to suppress the proliferative response of autologous CD4⁺ T cells in response to αCD3 mAbs cross-linked on T-cell-depleted PBMCs (8A) or immobilized on plastic (8B). Interestingly, only those clones which were anergic and displayed a constitutively high expression of CD25 had a suppressive phenotype. When data from all the CD25⁺CD4⁺ T-cell clones tested were pooled together, and the clones were separated into suppressive and non-suppressive groups, expression of CD25 was found to be significantly higher in the group with suppressive activity (p<0.000007) (Table 2). In contrast, proliferation in response to αCD3 mAbs was a less reliable predictor of suppressive capacity, as several clones within the non-suppressive category were anergic. These data indicate that constitutively high expression of CD25 is a marker for CD4⁺ T regulatory cells at the clonal level.

We also determined the cytokine production profile of the CD25⁺CD4⁺ T-cell clones. As shown in Table 3, non-suppressive clones tended to possess a Th0 pattern of cytokine production and produced moderate levels of most cytokines tested. In contrast, all CD25⁺CD4⁺ T-cell clones which had suppressive activity produced significant amounts of TGF-β, small and variable amounts of IL-4, IL-5 and IFN-γ, but failed to produce detectable levels of IL-2 or IL-10. These data indicate that CD25⁺CD4⁺ T regulatory cells are likely distinct from IL-10-producing Tr1 cells, although it cannot be excluded that they represent the same cells at different stages of differentiation. The fact that the only cytokine which was consistently detected in the supernatants of all the suppressive CD25⁺CD4⁺ T-cell clones was TGF-β suggests that they are more likely related to the TGF-β producing Th3 cells which were originally described in models of oral tolerance (27, 28).

### TABLES

**Table 1. Cytokine production by CD25⁺CD4⁺ Tr cells. Purified cells were stimulated as indicated and supernatants were collected after 24 (for IL-2) or 72 hours. The amount of cytokine was determined by ELISA. Data represent the average values (pg/ml) of pooled data from 4 independent experiments. Cytokine production by allogeneic APCs alone has been subtracted.**

| **Stimuli** | **Cells** | **IL-2** | **IL-4** | **IL-10** | **IFN-γ** | **TGF-β** |
|---|---|---|---|---|---|---|
| αCD3/28 +IL-2 | CD25⁺ | N.D. | 153 | 1148 | 5723 | 1322 |
| αCD3/28 +IL-2 | CD25 | N.D. | 94 | 840 | 9773 | 1225 |
| allogeneic APCs | CD25⁺ | <20 | <20 | 298 | 527 | 509 |
| allogeneic APCs | CD25 | 99.5 | <20 | 251 | 5744 | 637 |

**Table 2. Suppressive CD25⁺CD4⁺ T-cell clones have a distinct phenotype from non-suppressive clones. Summary of the phenotype of all the CD25⁺CD4⁺ T-cell clones which were extensively characterized. Percent suppression represents the average reduction of proliferation of autologous CD4⁺ T cells upon activation with αCD3 mAbs, immobilized on plastic or T-cell-depleted ACPs, in the presence of the indicated T-cell clones. Numbers represent the average suppression observed in 2-6 independent experiments. MFI represents the average expression of CD25 as determined in 2-6 independent tests. cpm represents the amount of thymidine incorporated following activation with αCD3 mAbs immobilized on plastic. Numbers represent the average of duplicates in a single test, and are representative of results obtained in several subsequent tests.**

| | **suppression (%)** | **CD25 MFI** | **αCD3 (cpm)** |
|---|---|---|---|
| ***non-suppressive*** | | | |
| 2 | 0 | 36 | 15947 |
| 3 | 0 | 83 | 166 |
| 6 | 0 | 40 | 908 |
| 37 | 0 | 40 | 36280 |
| 84 | n.t. | 19 | 20281 |
| 85 | 0 | 33 | 1839 |
| 86 | 0 | 91 | 23139 |
| 87 | 0 | 21 | 4166 |
| 88 | n.t. | 34 | 36376 |
| 89 | 0 | 43 | 17702 |
| 90 | 0 | 46 | 21210 |
| 92 | 0 | 33 | 34004 |
| 93 | n.t. | 19 | 20005 |
| 94 | n.t. | 12 | 6929 |
| 95 | 0 | 66 | 410 |
| ***suppressive*** | | | |
| 4 | 37 | 90 | 244 |
| 12 | 40 | 100 | 122 |
| 13 | 52 | 101 | 511 |
| 15 | 24 | 211 | 644 |
| 17 | 36 | 110 | 88 |

| | | | |
|---|---|---|---|
| 18 | 73 | 97 | 123 |
| 19 | 60 | 88 | 1289 |
| 20 | 22 | 98 | 285 |
| 21 | 76 | 65 | 72 |
| 22 | 39 | 102 | 180 |
| 24 | 45 | 174 | 281 |
| 28 | 45 | 179 | 380 |
| 29 | 54 | 113 | 98 |
| 40 | 63 | 85 | 108 |
| 42 | 45 | 77 | 52 |
| 47 | 57 | 86 | 61 |
| 48 | 56 | 68 | 66 |
| 57 | 31 | 130 | 600 |

| | | | |
|---|---|---|---|
| *Nt: not tested* | | | |

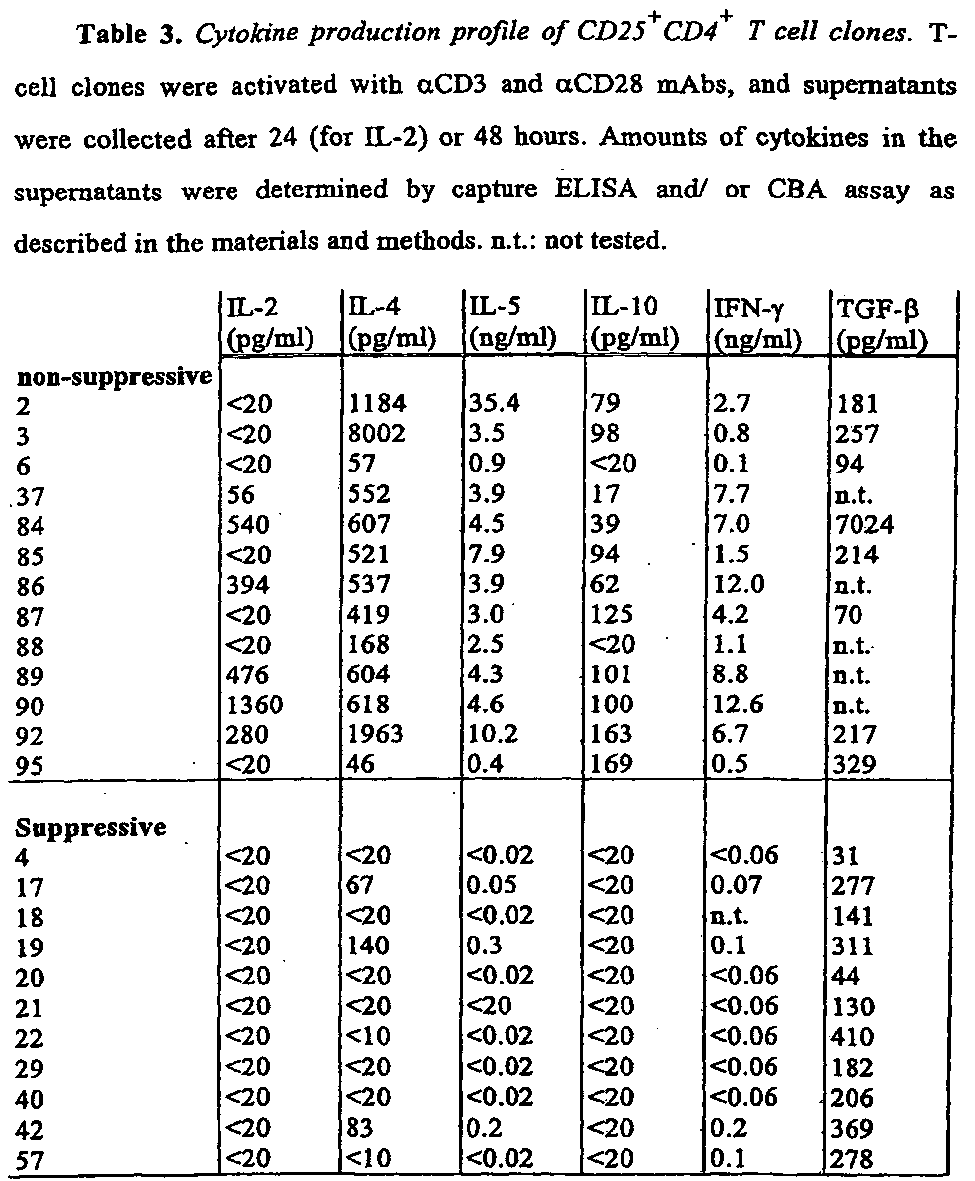

### MATERIALS AND METHODS

### 1. Isolation and characterization of human CD25⁺CD4⁺ Tr cells

*Purification of CD25*^{*+*}*CD4*^{*+*} *Tr cells.* Human peripheral blood was obtained from healthy donors in accordance with local ethical committee approval. PBMCs were prepared by centrifugation over Ficoll-Hypaque gradients (Nycomed Amersham, Uppsala, Sweden), and CD4⁺ T cells were purified by positive or negative selection (by depletion of CD8, CD11b, CD16, CD19, CD36 and CD56 positive cells) with the CD4⁺ Multisort kit or the Untouched CD4⁺ T cell Isolation kit, respectively (Miltenyi Biotech, Gladbach, Germany). Following isolation of CD4⁺ T cells, CD25⁺ cells were stained with PE-coupled anti-CD25 mAbs and purified following addition of anti-PE coupled magnetic beads (Miltenyi Biotech). Alternatively, CD4⁺ T cells were purified with magnetic beads directly coupled to anti-CD25 (Miltenyi Biotech) to facilitate FACS analysis. Results obtained with CD25⁺CD4⁺ Tr cells isolated by negative or positive selection, and directly or indirectly coupled CD25 mAbs were identical. Starting with 2x10⁸ PBMCs, typically 2-3x10⁶ CD25⁺CD4⁺ Tr cells were isolated, with a purity ranging from 90-95%. CD25⁻CD4⁺ T cells were also collected, with a purity ranging from 70-90%. For purification of CD25⁺ cells following *in vitro* activation of CD25⁻ cells, CD25⁻CD4⁺ T cells were activated for 48 hours with immobilized anti-CD3 (10µg/ml) and soluble anti-CD28 (1µg/ml) mAbs and CD25⁺ T cells were purified as described above.

*In vitro expansion of T cell lines.* CD25⁺CD4⁺ or CD25⁻CD4⁺ T cells were isolated as described. T cells (2x10⁵ cells/ml) were stimulated with anti-CD3 (1µg/ml) (OKT3, Orthoclone, Jansen Cilag, Italy) in the presence of an allogeneic feeder-cell mixture consisting of 1x10⁶ PBMCs/ml, (irradiated 6000 RADS) and 1x10⁵ JY cells/ml (irradiated 10,000 RADS), an EBV-LCL which expresses high levels of HLA and costimulatory molecules as well as cytokines, as previously described (29, 30). All cultures were performed in X-Vivo 15 medium (BioWhittaker, Bergamo, Italy) supplemented with 10% FCS (Mascia Brunelli, Milan, Italy), 1% pooled human serum, 100 U/ml penicillin/streptomycin (Bristol-Myers Squibb, Sermoneta, Italy) and 2mM glutamine (GibcoBRL, Milan, Italy) (hereafter referred to as complete medium). Three days after activation, 40U/ml rIL-2 (Chiron Italia, Milan, Italy) was added. Cells were split as necessary and fresh medium with IL-2 was added. T-cell lines were restimulated every 14 days. All experiments on expanded cells were performed at least 10 days after activation.

*Proliferation and suppression of T cells.* To analyze proliferation in response to polyclonal activation, 96 well round-bottom plates (Costar) were coated overnight at 4°C with anti-CD3 mAbs (10µg/ml) in 0.1M Tris, pH 9.5, and washed three times with PBS. T cells were plated at an initial density of 5x10⁵ cells/ml (100,000 cells/well) in a final volume of 200µl of complete medium in the absence or presence of soluble anti-CD28 mAbs (1µg/ml) (Pharmingen, San Diego, CA), soluble secondary rabbit anti-mouse Abs (10µg/ml) (Sigma, Milan, Italy) and/or IL-2 (100U/ml).

To test antigen-specific T cell proliferation, freshly isolated CD25⁺CD4⁺ Tr or CD25⁻CD4⁺ T cells (2.5x10⁵ cells/ml) were stimulated with irradiated (6000 Rads) allogeneic PBMCs (2.5x10⁵ cells/ml) that had been depleted of CD3⁺ cells by negative selection (Dynal, Oxoid). For suppression, increasing numbers (up to 2.5x10⁵ cells/ml) of freshly isolated autologous CD25⁺CD4⁺ Tr cells were added. Cells were co-cultured in a final volume of 200µl of complete medium in 96 well round-bottom plates. Control IgG (10µg/ml) (Pharmingen), neutralizing anti-IL-10R (10µg/ml) (3F9, Pharmigen) and/or anti-TGF-β_{1,2,3} (10µg/ml or 50µg/ml) (R&D), or F(ab')₂ anti-CTLA-4 (10µg/ml) (Ancell, Bayport, MN) mAbs were added as indicated. For suppression of memory T cells, cells from expanded CD25⁻CD4⁺ T-cell lines were cultured with allogeneic APCs (from a donor different from that used in the allogeneic feeder-cell mixture), and increasing numbers of expanded autologous CD25⁺CD4⁺ Tr cells were added as described above. For control experiments, CD25⁺CD4⁺ T cells purified from *in vitro*-activated CD25⁻CD4⁺ T cells were added in increasing numbers to freshly isolated autologous CD25⁻CD4⁺ T cells and allogeneic APCs.

After the indicated time, wells were pulsed for 16 hours with 1µCi/well ³H-thymidine (Amersham, Uppsala, Sweden). Cells were harvested, and counted in a scintillation counter.

*ELISAs*. For detection of IL-10, IL-4, IL-2, IFN-γ, and TGF-β, capture ELISAs were performed on supernatants of cells (1x10⁶ T cells/ml) that had been stimulated with immobilized anti-CD3 mAbs (10µg/ml) with or without anti-CD28 (1µg/ml) and IL-2 (100U/ml), or irradiated CD3-depleted allogeneic PBMCs (1x10⁶ cells/ml) for 24 (for IL-2) or 72 hours. ELISAs were performed according to the manufacturer's instructions. All capture and detection mAbs were purchased from Pharmingen. The limits of detection were as follows: IL-2: 19pg/ml; IL-4: 9.4pg/ml; IL-10: 15.6pg/ml; IFN-γ: 62.5pg/ml; TGF-β: 62.5 pg/ml.

*FACS analysis.* Anti-CD4, -CD25, -HLA-DR, -CD45RO, -CD62L, -CD69 and -CD40L mAbs were purchased from Beckton Dickinson (Mountain View, CA) and were directly coupled to FITC or PE. Expression of IL-2Rβ (CD122) and IL-2Rγ (CD132) was determined by staining with the relevant biotinylated mAbs (PharMingen) and streptavidin-coupled TriColor (Caltag). Cells that were resting, or that had been activated with immobilized anti-CD3 (10µg/ml), or PMA (10ng/ml, Sigma) and calcium ionophore (A23187, 500ng/ml, Sigma), were incubated with the indicated mAbs for 20 mins at 4°C in PBS, 2% FCS, washed once and analyzed with a FACScan® flowcytometer using Cellquest software (Beckton Dickinson). Expression of CTLA-4 was determined by intracytoplasmic staining with biotinylated anti-CTLA-4 (PharMingen) followed by streptavidin-coupled PE (Caltag). Resting or activated cells were fixed with 2% formaldehyde, and membranes were permeabilized by incubation in saponin buffer (PBS, 2% BSA and 0.5% saponin (Sigma)) for 10 mins. Staining and washing were performed in saponin buffer, and cells were washed once in PBS, 2% BSA prior to analysis.

### 2. Isolation and characterization of human CD25⁺CD4⁺ Tr cell clones

*Purification and cloning of CD25*^{*+*}*CD4*^{*+*} *Tr cells.* CD4⁺ T cells from PBMCs were obtained as described above. CD4⁺ T cells were stained with FITC-coupled anti-CD4 and PE-coupled anti-CD25 mAbs (Beckton-Dickson) and CD25⁺ and CD25⁻ cells were purified by FACS-sorting on a FACStar (Beckton-Dickson). CD25⁺CD4⁺ and CD25⁻CD4⁺ T cells were subsequently cloned at 1 cell/well in 96-well round bottom plates by limiting dilution in the presence of an allogeneic feeder-cell mixture consisting of 5x10⁵ PBMCs/ml, 5x10⁴ JY cells/ml and 0.05µg/ml PHA. After 3 days, IL-2 (40 U/ml) was added. Tcell clones were cultured in X-vivo 15 with 5% Human Serum. At day 14, growing wells were picked and re-stimulated with an allogeneic feeder-cell mixture as described above. Clones were split as necessary, and restimulated as above every 14 days. The medium was replenished every 3-5 days. Clones were used for experiments between days 10 and 14 of the previous re-stimulation (i.e. in the resting phase).

*Proliferation and suppression of T cells.* To analyze the proliferative capacity of T-cell clones in response to polyclonal activation, 96 well round-bottom plates (Costar) were coated overnight at 4°C with anti-CD3 mAbs (10µg/ml) in 0.1M Tris, pH 9.5, and washed three times with PBS. T-cell clones were plated at an initial density of 2x10⁵ cells/ml (40,000 cells/well) in a final volume of 200µl of complete medium in the absence or presence of IL-2 (100U/ml). To test for the capacity of T-cell clones to suppress the proliferation of autologous CD4⁺ T cells, fresh CD4⁺ T cells were purified by positive selection (Miltenyi Biotech) and stimulated with anti-CD3 mAbs which had been immobilized on plastic (as described above) or bound to allogeneic CD3-depleted PBMCs (irradiated 6000 RADS). CD4⁺ T cells (40,000 cells/well) were cultured alone, or in the presence of a 1:1 ratio of T-cell clones in a final volume of 200µl of complete medium in 96 well round-bottom plates.

After the indicated time, wells were pulsed for 16 hours with 1µCi/well 3H-thymidine (Amersham, Uppsala, Sweden). Cells were harvested, and counted in a scintillation counter.

*ELISAs*. T cell clones (1x10⁶ cells/ml) were stimulated with immobilized anti-CD3 mAbs (10µg/ml) and anti-CD28 (1µg/ml), and supernatants were collected after 24 hours for IL-2 and after 48 hours for all other cytokines. Levels of TGF-β in acidified supernatants were determined by capture ELISA as described above. Levels of IL-2, IL-4, IL-5, IL-10 and IFN-γ were either determined either by capture ELISA (BD Biosciences) as described above or by the cytometric bead array kit (CBA) (BD Biosciences), according to the manufacture's instructions. A direct comparison of capture ELISA and CBA demonstrated that the two methods were highly comparable in terms of the amount of cytokine detected in the supernatant.

*Statistical Analysis*. All analysis for statistically significant differences were performed with the student's paired *t* test. *p* values less than 0.05 were considered significant. Results are expressed as means ± SEM.

### Abbreviations

- CD: cluster of differentiation
- IL: interleukin
- TGF: transforming growth factor
- APC: antigen presenting cell
- mAb: monoclonal antibody
- FACS: fluorescence activated cell sorting
- PHA: phytohemoagglutinin
- PBMC: peripheral blood mononuclear cells
- MFI: mean fluorescence intensity
- EBV: Ebstein-Barr virus

### REFERENCES

1. Roncarolo, M.G., and M.K. Levings. 2000. The role of different subsets of T regulatory cells in controlling autoimmunity. *Curr. Opin. Immunol.* 12:676-83.
2. Sakaguchi, S. 2000. Regulatory T cells: Key Controllers of Immunologic Self-Tolerance. *Cell* 101:455-8.
3. Shevach, E.M. 2000. Regulatory T cells in autoimmmunity. *Annu Rev Immunol* 18:423-49.
4. Cottrez, F., S.D. Hurst, R.L. Coffman, and H. Groux. 2000. T regulatory cells 1 inhibit a Th2-specific response in vivo. *J. Immunol.* 165:4848-53.
5. Asseman, C., S. Mauze, M.W. Leach, R.L. Coffman, and F. Powrie. 1999. An essential role for interleukin 10 in the function of regulatory T cells that inhibit intestinal inflammation. *J Exp Med* 190, no. 7:995-1004.
6. Groux, H., A. O'Garra, M. Bigler, M. Rouleau, S. Antonenko, J.E. de Vries, and M.G. Roncarolo. 1997. A CD4⁺ T-cell subset inhibits antigen-specific T-cell responses and prevents colitis. *Nature* 389, no. 6652:737-42.
7. Powrie, F., J. Carlino, M.W. Leach, S. Mauze, and R.L. Coffman. 1996. A critical role for transforming growth factor-beta but not interleukin 4 in the suppression of T helper type 1-mediated colitis by CD45RB(low) CD4⁺ T cells. *J Exp Med* 183, no. 6:2669-74.
8. Chen, Y., V.K. Kuchroo, J. Inobe, D.A. Hafler, and H.L. Weiner. 1994. Regulatory T cell clones induced by oral tolerance: suppression of autoimmune encephalomyelitis. *Science* 265, no. 5176:1237-40.
9. Salomon, B., D.J. Lenschow, L. Rhee, N. Ashourian, B. Singh, A. Sharpe, and J.A. Bluestone. 2000. B7/CD28 costimulation is essential for the homeostasis of the CD4⁺CD25⁺ immunoregulatory T cells that control autoimmune diabetes. *Immunity* 12, no. 4:431-40.
10. Read, S., V. Malmstrom, and F. Powrie. 2000. Cytotoxic T Lymphocyte-associated Antigen 4 Plays an Essential Role in the Function of CD25(+)CD4(+) Regulatory Cells that Control Intestinal Inflammation. *J Exp Med* 192, no. 2:295-302.
11. Suri-Payer, E., A.Z. Amar, A.M. Thornton, and E.M. Shevach. 1998. CD4⁺CD25⁺ T cells inhibit both the induction and effector function of autoreactive T cells and represent a unique lineage of immunoregulatory cells. *J Immunol* 160, no. 3:1212-8.
12. Asano, M., M. Toda, N. Sakaguchi, and S. Sakaguchi. 1996. Autoimmune disease as a consequence of developmental abnormality of a T cell subpopulation. *J Exp Med* 184:3 87-96.
13. Itoh, M., T. Takahashi, N. Sakaguchi, Y. Kuniyasu, J. Shimizu, F. Otsuka, and S. Sakaguchi. 1999. Thymus and autoimmunity: production of CD25⁺CD4⁺ naturally anergic and suppressive T cells as a key function of the thymus in maintaining immunologic self-tolerance. *J Immunol* 162, no. 9:5317-26.
14. Sakaguchi, S., N. Sakaguchi, M. Asano, M. Itoh, and M. Toda. 1995. Immunologic self-tolerance maintained by activated T cells expressing IL-2 receptor alpha-chains (CD25). Breakdown of a single mechanism of self-tolerance causes various autoimmune diseases. *J Immunol* 155:1151-64.
15. Thornton, A.M., and E.M. Shevach. 1998. CD4⁺CD25⁺ immunoregulatory T cells suppress polyclonal T cell activation in vitro by inhibiting interleukin 2 production. *J Exp Med* 188, no. 2:287-96.
16. Takahashi, T., Y. Kuniyasu, M. Toda, N. Sakaguchi, M. Itoh, M. Iwata, J. Shimizu, and S. Sakaguchi. 1998. Immunologic self-tolerance maintained by CD25⁺CD4⁺ naturally anergic and suppressive T cells: induction of autoimmune disease by breaking their anergic/suppressive state. *Int Immunol* 10, no. 12:1969-80.
17. Thornton, A.M., and E.M. Shevach. 2000. Suppressor effector function of CD4⁺CD25⁺ immunoregulatory T cells is antigen nonspecific. *J Immunol* 164, no. 1:183-90.
18. Takahashi, T., T. Tagami, S. Yamazaki, T. Uede, J. Shimizu, N. Sakaguchi, T.W. Mak, and S. Sakaguchi. 2000. Immunologic Self-Tolerance Maintained by CD25(+)CD4(+) Regulatory T Cells Constitutively Expressing Cytotoxic T Lymphocyte-associated Antigen 4. *J Exp Med* 192, no. 2:303-310.
19. Cederbom, L., H. Hall, and F. Ivars. 2000. CD4⁺CD25⁺ regulatory T cells down-regulate co-stimulatory molecules on antigen-presenting cells. *Eur J Immunol* 30:1538-43.
20. Kitani, A., K. Chua, K. Nakamura, and W. Strober. 2000. Activated Self-MHC-Reactive T Cells Have the Cytokine Phenotype of Th3/T Regulatory Cell 1 T Cells. *J Immunol* 165, no. 2:691-702.
21. Kuniyasu, K., T. Takahashi, M. Itoh, J. Shimizu, G. Toda, and S. Sakaguchi. 2000. Naturally anergic and suppressive CD25(+)CD4(+) T cells as a functionally and phenotypically distinct immunoregulatory T cell population. *Int Immunol* 12:1145-55.
22. Roncarolo, M.G., R. Bacchetta, C. Bordignon, S. Narula, and M.K. Levings. 2001. Type 1 T regulatory cells. *Immunol Rev* 182:68-79.
23. Shevach, E.M., R.S. McHugh, C.A. Piccirillo, and A.M. Thornton. 2001. Control of T-cell activation by CD4⁺ CD25⁺ suppressor T cells. *Immunol Rev* 182:58-67.
24. Sakaguchi, S., N. Sakaguchi, J. Shimizu, S. Yamazaki, T. Sakihama, M. Itoh, Y. Kuniyasu, T. Nomura, M. Toda, and T. Takahashi. 2001. Immunologic tolerance maintained by CD25⁺ CD4⁺ regulatory T cells: their common role in controlling autoimmunity, tumor immunity, and transplantation tolerance. *Immunol Rev* 182:18-32.
25. Baecher-Allan, C., J.A. Brown, G.J. Freeman, and D.A. Hafler. 2001. CD4⁺CD25high regulatory cells in human peripheral blood. *J Immunol* 167, no. 3:1245-53.
26. Levings, M.K., R. Sangregorio, and M.G. Roncarolo. 2001. Human CD25⁺CD4⁺ T regulatory cells suppress naive and memory T-cell proliferation and can be expanded *in vitro* without loss of function. *J. Exp. Med.* 193:1295-1302.
27. Chen, Y., J. Inobe, and H.L. Weiner. 1995. Induction of oral tolerance to myelin basic protein in CD8-depleted mice: both CD4⁺ and CD8⁺ cells mediate active suppression. *J Immunol* 155, no. 2:910-6.
28. Weiner, H.L. 2001. Induction and mechanism of action of transforming growth factor-beta- secreting Th3 regulatory cells. *Immunol Rev* 182:207-14.
29. Bacchetta, R., M. Bigler, J.L. Touraine, R. Parkman, P.A. Tovo, J. Abrams, R. de Waal Malefyt, J.E. de Vries, and M.G. Roncarolo. 1994. High levels of interleukin 10 production in vivo are associated with tolerance in SCID patients transplanted with HLA mismatched hematopoietic stem cells. *J Exp Med* 179, no. 2:493-502.
30. van de Griend, R.J., and R.L. Bolhuis. 1984. Rapid expansion of allospecific cytotoxic T cell clones using nonspecific feeder cell lines without further addition of exogenous IL2. *Transplantation* 38:401-6.
31. Taylor PA, Lees CJ, Blazar BR. The infusion of ex-vivo activated and expanded CD4(+)CD25(+) immune regulatory cells inhibits graft-versus-host disease lethality. Blood 2002 May 15; 99(10): 3493-9
32. Gould DS, Auchincloss H Jr. Direct and indirect recognition: the role of MHC antigens in graft rejection. Immunol. Today, 1999 Feb; 20(2):77-82.

## Claims

1. The use of ex-vivo isolated and expanded human CD25⁺CD4⁺ Tr cells for the preparation of immunomodulating or immunosuppressive agents.

2. The use of ex-vivo isolated human CD25⁺CD4⁺ Tr cell clones constitutively expressing CD25 for the preparation of immunomodulating or immunosuppressive agents.

3. The use according to claim 1 or 2, for the prevention or therapy of graft-vs-host disease, organ rejection, autoimmune diseases and for the prevention of adverse immune responses to transgenes and vector-derived proteins after gene therapy.

4. The use according to claim 1, wherein human CD25⁺CD4⁺ Tr cells are expanded in vitro under one or more of the following conditions: co-culture with feeder-cell mixture, polyclonal stimulation, antigen specific stimulation, addition of cytokines.

5. The use according to claim 2, wherein the CD25⁺CD4⁺ Tr cell clones constitutively expressing CD25 are isolated ex-vivo by the following steps:
a) purifying CD4⁺T cells from PBMCs;
b) separating CD25⁺ from CD25⁻T cells;
c) cloning CD25⁺CD4⁺ T cells by limiting dilution;
d) stimulation with phytohemagglutinin or and-CD3 mAb, in the presence of IL-2;
e) selecting the suppressive clones that display a constitutively high expression of CD25.

6. An immunosuppressive agent containing ex-vivo expanded human CD25⁺CD4⁺ Tr cells or isolated CD25⁺CD4⁺ Tr cell clones constitutively expressing CD25.

7. An immunosuppressive agent according to claim 6, further containing cytokines or additional immunosuppressants.

8. An immunosuppressive agent according to claims 6-7, which is in form of stabilized cell preparation.

9. A method of isolating immunosuppressive CD25⁺CD4⁺ Tr cell clones which comprises the steps of:
a) purifying CD4⁺T cells from PBMCs;
b) separating CD25⁺ from CD25⁻ T cells;
c) cloning CD25⁺CD4⁺ T cells by limiting dilution;
d) stimulation with phytohemagglutinin or anti-CD3 mAb, in the presence of IL-2;
c) selecting the suppressive clones that display a constitutively high expression of CD25.

10. A method according to claim 9, wherein the stimulation according to step (d) is carried out in the presence of an allogenic or autologous feeder-cell mixture consisting of irradiated PBMCs.

11. A method according to claim 10, which is carried out with irradiated autologous or allogeneic EBV-transformed cell lines.

12. A method according to claim 9, wherein in step (d) the suppressive clones are selected on the basis of the following characteristics:
- 100% constant-positivity for CD25 expression in the resting phase at least 10 days after stimulation with phytohemagglutinin or anti-CD3 mAb in the presence of IL-2;
- expression of CD25 at a significantly higher level in comparison to T cell clones isolated in parallel from CD25⁻CD4⁺ T cells or non suppressive clones isolated from CD25⁺CD4⁺ T cells.

13. Isolated CD25⁺CD4⁺ Tr cell clones obtainable by the process of claims 9-12.

14. Isolated CD25⁺CD4⁺ Tr cell clones according to claim 13, which do not produce IL-2.

15. The use of CD25⁺CD4⁺ Tr cell clones according to claims 13-14 for the preparation of in vitro systems for the identification of molecules that modulate the immune response.

16. The use according to claim 15, in large scale gene expression arrays, differential proteornies screenings and for the generation of monoclonal antibodies.

## Patentansprüche

1. Verwendung von *ex vivo* isolierten und expandierten humanen CD25⁺CD4⁺-Tr-Zellen zur Herstellung von Immunmodulatoren oder Immunsuppressiva.

2. Verwendung von *ex vivo* isolierten humanen CD25⁺CD4⁺-Tr-Zellklonen, welche konstitutiv CD25 exprimieren, zur Herstellung von Immunmodulatoren oder Immunsuppressiva.

3. Verwendung nach Anspruch 1 oder 2 zur Verhütung oder Therapie von Transplantat-Wirt-Abstoßung, Organabstoßung, Autoimmunerkrankungen und zur Verhütung negativer Immunreaktionen auf Transgene und Vektor-abgeleitete Proteine nach einer Gentherapie.

4. Verwendung nach Anspruch 1, wobei humane CD25⁺CD4⁺-Tr-Zellen *in vitro* unter einer oder mehreren der folgenden Bedingungen expandiert werden: Cokultur mit einer Feederzell-Mischung, polyklonale Stimulation antigenspezifische Stimulation, Zugabe von Zytokinen.

5. Verwendung nach Anspruch 2, wobei die CD25⁺CD4⁺-Tr-Zellklone, die konstitutiv CD25 exprimieren, *ex vivo* mit den folgenden Schritten isoliert werden:
a) Reinigung von CD4⁺-T-Zellen aus PBMCs;
b) Abtrennen von CD25⁺- von CD25⁻-T-Zellen;
c) Klonierung von CD25⁺CD4⁺-T-Zellen durch Grenzverdünnung;
d) Stimulation mit Phytohämagglutinin oder Anti-CD3-mAb, in Gegenwart von IL-2;
e) Selektion der suppressiven Klone, welche eine konstitutiv hohe Expression von CD25 zeigen.

6. Immunsuppressivum, enthaltend *ex vivo* expandierte humane CD25⁺CD4⁺-Tr-Zellen oder isolierte CD25⁺CD4⁺-Tr-Zellklone, die konstitutiv CD25 exprimieren.

7. Immunsuppressivum nach Anspruch 6, ferner enthaltend Zytokine oder zusätzliche Immunsuppressiva.

8. Immunsuppressivum nach den Ansprüchen 6-7, welches in Form einer stabilisierten Zellpräparation vorliegt.

9. Verfahren zur Isolierung von immunsuppressiven CD25⁺CD4⁺-Tr-Zellklonen, umfassend die Schritte:
a) Reinigung von CD4⁺-T-Zellen aus PBMCs;
b) Abtrennen von CD25⁺- von CD25⁻-T-Zellen;
c) Klonierung von CD25⁺CD4⁺-T-Zellen durch Grenzverdünnung;
d) Stimulation mit Phytohämagglutinin oder Anti-CD3-mAb, in Gegenwart von IL-2;
e) Selektion der suppressiven Klone, welche eine konstitutiv hohe Expression von CD25 zeigen.

10. Verfahren nach Anspruch 9, wobei die Stimulation gemäß Schritt (d) in Gegenwart einer allogenen oder autologen Feederzell-Mischung, bestehend aus bestrahlten PBMCs, durchgeführt wird.

11. Verfahren nach Anspruch 10, welches mit bestrahlten autologen oder allogenen EBV-transformierten Zelllinien durchgeführt wird.

12. Verfahren nach Anspruch 9, wobei in Schritt (d) die suppressiven Klone auf Basis der folgenden charakteristischen Merkmale ausgewählt werden:
- 100 % konstante Positivität für CD25-Expression in der Ruhephase mindestens 10 Tage nach Stimulation mit Phytohämagglutinin oder Anti-CD3-mAb in Gegenwart von IL-2;
- Expression von CD25 auf einem signifikant höheren Niveau im Vergleich zu T-Zellklonen, die parallel aus CD25-CD4⁺-T-Zellen isoliert wurden, oder nicht-suppressiven Klonen, die aus CD25⁺CD4⁺-T-Zellen isoliert wurden.

13. Isolierte CD25⁺CD4⁺-Tr-Zellklone, erhältlich mit dem Verfahren der Ansprüche 9 - 12.

14. Isolierte CD25⁺CD4⁺-Tr- Zellklone nach Anspruch 13, welche kein IL-2 produzieren.

15. Verwendung von CD25⁺CD4⁺-Tr-Zellklonen nach den Ansprüchen 13-14 zur Herstellung von *in vitro*-Systemen für die Identifizierung von Molekülen, welche die Immunreaktion modulieren.

16. Verwendung nach Anspruch 15 in Genexpressions-Arrays in großem Maßstab, differentiellen proteonomischen Screenings und zur Herstellung monoklonaler Antikörper.

## Revendications

1. Utilisation de cellules Tr CD25⁺CD4⁺ humaines isolées ex vivo et multipliées pour la préparation d'agents immunomodulateurs ou immunosuppressifs.

2. Utilisation de clones de cellules Tr CD25⁺CD4⁺ humaines isolés ex vivo, exprimant de manière constitutive CD25 pour la préparation d'agents immunomodulateurs ou immunosuppressifs.

3. Utilisation selon la revendication 1 ou 2, pour la prévention ou la thérapie de la maladie du greffon contre l'hôte, du rejet d'organe, des maladies auto-immunes ou pour la prévention des réponses immunitaires néfastes contre les transgènes et les protéines dérivées de vecteurs après une thérapie génique.

4. Utilisation selon la revendication 1, dans laquelle les cellules Tr CD25⁺CD4⁺ humaines sont multipliées in vitro dans une ou plusieurs des conditions suivantes: co-culture avec un mélange de cellules nourricières, stimulation polyclonale, stimulation spécifique d'antigène, addition de cytokines.

5. Utilisation selon la revendication 2, dans laquelle les clones de cellules Tr CD25⁺CD4⁺ exprimant de manière constitutive CD25 sont isolés ex vivo suivant les étapes suivantes:
a) purification des cellules T CD4⁺ à partir de PBMC;
b) séparation de CD25⁺ des cellules T CD25⁻;
c) clonage des cellules T CD25⁺CD4⁺ par dilution limitante;
d) stimulation avec de la phytohémagglutinine ou du mAb anti-CD3, en présence de IL-2;
e) sélection des clones suppressifs qui présentent une expression constitutivement élevée de CD25.

6. Agent immunosuppressif contenant des cellules Tr CD25⁺CD4⁺ humaines multipliées ex vivo ou des clones de cellules Tr CD25⁺CD4⁺ isolés, exprimant de manière constitutive CD25.

7. Agent immunosuppressif selon la revendication 6, contenant en outre des cytokines ou des immunosuppresseurs supplémentaires.

8. Agent immunosuppressif selon les revendications 6 et 7, qui est sous forme de préparation cellulaire stabilisée.

9. Procédé d'isolement de clones de cellules Tr CD25⁺CD4⁺ immunosuppressifs, qui comprend les étapes de:
a) purification des cellules T CD4⁺ à partir de PBMC;
b) séparation de CD25⁺ des cellules T CD25⁻;
c) clonage des cellules T CD25⁺CD4⁺ par dilution limitante;
d) stimulation avec de la phytohémagglutinine ou du mAb anti-CD3, en présence de IL-2;
e) sélection des clones suppressifs qui présentent une expression constitutivement élevée de CD25.

10. Procédé selon la revendication 9, dans lequel la stimulation selon l'étape (d) est réalisée en présence d'un mélange de cellules nourricières allogéniques ou autologues constitué de PBMC irradiées.

11. Procédé selon la revendication 10, qui est mis en oeuvre avec des lignées de cellules transformées par EBV, autologues ou allogéniques irradiées.

12. Procédé selon la revendication 9, dans lequel dans l'étape (d), les clones suppressifs sont choisis sur la base des caractéristiques suivantes:
- un taux de positivité constant de 100% de l'expression de CD25 dans la phase de repos au moins 10 jours après la stimulation avec la phytohémagglutinine ou le mAb anti-CD3 en présence de IL-2;
- une expression de CD25 à un niveau nettement supérieur par rapport à des clones de cellules T isolés en parallèle à partir de cellules T CD25CD4⁺ ou des clones non suppressifs isolés à partir de cellules T CD25⁺CD4⁺.

13. Clones de cellules Tr CD25⁺CD4⁺ isolés, pouvant être obtenus par le procédé des revendications 9 à 12.

14. Clones de cellules Tr CD25⁺CD4⁺ isolés selon la revendication 13, qui ne produisent pas de IL-2.

15. Utilisation de clones de cellules Tr CD25⁺CD4⁺ selon les revendications 13 et 14 pour la préparation de systèmes in vitro pour l'identification de molécules qui modulent la réponse immunitaire.

16. Utilisation selon la revendication 15, dans des panoplies d'expression génique à grande échelle, des criblages par protéomique différentielle et pour la production d'anticorps monoclonaux.
